# FASCICULE DE BREVET EUROPEEN

(11) **EP 2 491 136 B1**
(45) Date de publication et mention de la délivrance du brevet: **11.03.2015**
(21) Numéro de dépôt: 10774284.3
(22) Date de dépôt: 21.10.2010
(51) Int. Cl.: C12Q 1/68

(54) **TEST DE DÉTECTION DE XANTHOMONAS AXONOPODIS PV. ALLII**
TEST FÜR DEN NACHWEIS VON XANTHOMONAS AXONOPODIS PV. ALLII
TEST FOR DETECTING XANTHOMONAS AXONOPODIS PV. ALLII

(30) Priorité: 21.10.2009 FR 0905053
(43) Date de publication de la demande: 29.08.2012
(73) Titulaire: Centre de Cooperation Internationale en Recherche Agronomique pour le Developpement, 75016 Paris (FR)
(72) Inventeur: ROBENE, Isabelle, F-97434 Saint Gilles les Bains (FR); PRUVOST, Olivier, F-97430 Tampon (FR); LEGRAND, Delphine, F-30240 Le Grau du Roi (FR)
(74) Mandataire: Bernstein, Claire Jacqueline
(86) Numéro de dépôt international: PCT/IB2010/054774
(87) Numéro de publication internationale: WO 2011/048569

(56) Documents cités:
- PICARD Y ET AL: "Polyphasic characterization of Xanthomonas axonopodis pv. allii associated with outbreaks of bacterial blight on three Allium species in the Mascarene archipelago", PHYTOPATHOLOGY, vol. 98, no. 8, août 2008 (2008-08), pages 919-925, XP002577941, ISSN: 0031-949X cité dans la demande
- DATABASE EMBL [Online] 8 juillet 1993 (1993-07-08), "Xanthomonas campestris vesicatoria avirulence protein (avrRxv) gene, complete cds.", XP002577944, extrait de EBI accession no. EMBL:L20423 Database accession no. L20423
- RADEMAKER JAN L W ET AL: "Comparison of AFLP and rep-PCR genomic fingerprinting with DNA-DNA homology studies: Xanthomonas as a model system", INTERNATIONAL JOURNAL OF SYSTEMATIC AND EVOLUTIONARY MICROBIOLOGY, vol. 50, no. 6 part 2, mars 2000 (2000-03) , pages 665-677, XP002577943, ISSN: 1466-5026
- WHALEN M C ET AL: "Identification of a host 14-3-3 protein that interacts with Xanthomonas effector AvrRxv", PHYSIOLOGICAL AND MOLECULAR PLANT PATHOLOGY, ACADEMIC PRESS LTD, GB LNKD- DOI:10.1016/J.PMPP.2008.05.006, vol. 72, no. 1-3, 1 janvier 2008 (2008-01-01), pages 46-55, XP025407961, ISSN: 0885-5765 [extrait le 2008-07-17]
- BONSHTIEN ARALE ET AL: "Molecular properties of the Xanthomonas AvrRxv effector and global transcriptional changes determined by its expression in resistant tomato plants", MOLECULAR PLANT-MICROBE INTERACTIONS, vol. 18, no. 4, avril 2005 (2005-04), pages 300-310, XP002577942, ISSN: 0894-0282

## Description

La présente invention se rapporte à de nouveaux outils de détection de *Xanthornaonas axonopodis* pv. *allii.*

*Xanthornaonas axonopodis* pv. *allii* est l'agent causal du dépérissement bactérien de l'oignon, une des contraintes sanitaires majeures de la culture des alliacées. Cette maladie a été observée pour la première fois en 1971 à la Barbade (Antilles), puis en 1975 dans plusieurs îles de l'archipel d'Hawaii. Dans les années 1980 cette bactérie a été trouvée au Brésil, à Cuba et à l'île Maurice. De 1990 à 2000, elle a atteint les Etats-Unis, le Venezuela, l'Afrique du Sud et le Japon, provoquant des dégâts importants. *Xanthornaonas axonopodis* pv. *allii* a été caractérisée pour la première fois à Hawaii en 1978 (Alvarez et al., Physiopathology ; 68 : 1132-1136, 1978).

*Xanthornaonas axonopodis* pv. *allii* est pathogène des alliacées, notamment l'ail, le poireau, la ciboulette, l'échalotte et l'oignon (Roumagnac et al., Int. J. Syst. Evol. Microbiol., 54 : 15-24, 2004), la maladie tendant à être plus sévère chez l'oignon. Cette bactérie s'est également révélée pathogène pour des espèces du genre *Citrus* lors d'infections expérimentales par infiltration (Gent et al., Phytopathology, 95(8) : 918-925, 2005) ; il n'y a cependant aucune preuve montrant que cette bactérie est pathogène au champ pour le genre *Citrus.*

*Xanthornaonas axonopodis* pv. *allii* provoque chez l'oignon des lésions sur les tissus aériens de la plante (feuilles et hampes florales), caractérisées par la présence de petites tâches aqueuses lenticulaires qui s'étendent et évoluent rapidement en chloroses puis nécroses. La maladie est favorisée par des températures élevées (supérieures à 27 °C) et des foyers importants apparaissent rapidement (7 à 10 jours) après une période de temps humide et pluvieux. A cause de la réduction du feuillage, les plantes se rabougrissent et les bulbes s'amoindrissent, ce qui entraîne des pertes de rendement important, de l'ordre de 10 à 50 %. Par exemple, aux Etats-Unis, des pertes de rendement de 20 % ou plus sont couramment observées dans les champs infectés.

Il a été montré que la bactérie pouvait être présente dans les semences d'oignons (Roumagnac et al., Eur. J. Plant Pathol., 106 : 867-877, 2000), que la maladie pouvait se diffuser à large échelle, notamment par les échanges commerciaux de semences, et que 4 graines infectées sur 10000 suffisaient pour déclencher une épidémie (Roumagnac et al., Phytophatology, 94 : 138-146, 2004).

Au sein des cultures, le vent et l'irrigation, en particulier l'irrigation par aspersion, peuvent assurer une plus grande dissémination de la maladie, comme c'est également le cas avec les orages et la grêle. En outre, la bactérie est capable de survivre sur des débris de cultures qui sont ensuite disséminés en adhérant aux vêtements des travailleurs et aux équipements.

Du fait de l'importance des épidémies et de la faible proportion de graines contaminées nécessaire pour déclencher des épidémies conséquentes, cette bactérie a été mise sur la liste d'alerte de l'Organisation Européenne de la Protection des Plantes (OEPP) en 2006. Une procédure est en cours pour son inscription sur la liste A1 des organismes de quarantaine OEPP.

Des mesures de lutte contre le dépérissement bactérien de l'oignon sont disponibles, par exemple l'utilisation de semences saines, la destruction des repousses d'oignons, la destruction des débris végétaux, les rotations, la lutte chimique.

En outre, les commercialisations et échanges internationaux de semences sont les agents principaux de la diffusion de cette maladie et représentent un risque d'introduction de l'agent pathogène dans des zones saines ou d'introduction de nouveaux génotypes dans des zones déjà contaminées. Il est donc nécessaire de pouvoir garantir la bonne qualité sanitaire de ces semences. Pour ce faire, des tests de diagnostic rapides, fiables et sensibles permettant la certification des lots de semences d'*Allium* en routine sont indispensables. A l'heure actuelle de tels outils diagnostic n'existent pas. Les méthodes d'identification couramment utilisées sont basées sur l'isolement de la bactérie à partir du matériel végétal infecté et sa mise en culture sur un milieu approprié, notamment le milieu NCTM1 (Roumagnac et al., Eur. J. Plant Pathol., 106 : 867-877, 2000). L'isolement de la bactérie prend plusieurs jours, et l'identité de la bactérie doit ensuite être vérifiée par des méthodes biochimiques, des typages moléculaires et/ou tests de pathogénicité (Gent et al., Phytopathology, 94(2) : 184-195, 2004 ; Picard et al., Phytopathology, 98(9) : 919-925, 2008). De plus, la présence d'autres bactéries dans les échantillons peut dans certains cas diminuer la sensibilité du test et provoquer la détection de faux-positifs.

Un test d'identification par amplification PCR d'une séquence spécifique de *Xanthornaonas axonopodis* pv. *allii* a été développé (Humeau et al., Phytopathology, 96(12) : 1345-1354, 2006 ; Picard et al., Phytopathology, 98(9) : 919-925, 2008). Ce test consiste à réaliser une PCR nichée en utilisant lors d'une première étape d'amplification les amorces PXaa1U (SEQ ID NO : 1) / PXaa1L (SEQ ID NO : 2), puis dans une seconde étape les amorces NXaa1U (SEQ ID NO : 3) / NXaa1L (SEQ ID NO : 4). Cependant, bien que cette méthode soit sensible et fiable, elle ne permet pas la détection de toutes les souches, notamment celles provenant de la Barbade et du Brésil. Le défaut de détection de certaines souches s'expliquerait par la forte diversité génétique intra-pathovar chez *Xanthomonas axonopodis* pv. *allii* (Picard et al., Phytopathology, 98(9) : 919-925, 2008 ; Gent et al., Phytopathology, 94(2) : 184-195, 2004 ; Gent et al., Phytopathology, 95(8) : 918-925, 2005). En outre, une étude de diversité génétique a montré que le polymorphisme des souches était variable selon leur origine géographique : il existe des zones à forte diversité (notamment Etats-Unis, Afrique du Sud) et des zones à faible diversité (par exemple Venezuela, Hawaï, Barbade).

Par conséquent, puisqu'aucun test n'est aujourd'hui disponible pour détecter l'ensemble des souches de *Xanthomonas axonopodis* pv. *allii,* la mise au point d'un outil diagnostic fiable, sensible et permettant la détection de toutes les souches, quelle que soit leur origine géographique, est indispensable pour optimiser la lutte contre les maladies provoquées par cette bactérie et améliorer la surveillance sanitaire lors des échanges internationaux.

Les Inventeurs ont maintenant identifié deux séquences d'ADN cibles spécifiques de *Xanthornaonas axonopodis* pv. *allii.* Ces séquences cibles, nommées « PIL » et « AVR », sont représentées dans le listage de séquences en annexe sous les numéros SEQ ID NO: 5 et SEQ ID NO: 6, respectivement.

Le marqueur PIL, mis en évidence par la technique d'amplification aléatoire d'ADN polymorphe, ou RAPD pour « Random Amplification of Polymorphic DNA », est présent dans environ 70% des souches de *Xanthornaonas axonopodis* pv. *allii.* Il s'est avéré que les amorces PXaa1U (SEQ ID NO : 1) / PXaa1L (SEQ ID NO : 2) et NXaa1U (SEQ ID NO : 3) / NXaa1L (SEQ ID NO : 4) utilisées par Humeau et al. (Phytopathology, 96 : 1345-1354, 2006) s'hybridaient à cette séquence cible.

Le marqueur « AVR », identifié par la méthode AFLP (pour Amplified Fragment Length Polymorphism), est au moins présent chez toutes les souches qui ne possèdent pas le marqueur « PIL ».

Ainsi, du fait de la complémentarité de ces marqueurs, leur recherche conjointe permet le diagnostic sélectif de l'ensemble des souches connues de *Xanthomonas axonopodis* pv. *allii.*

La présente invention a donc pour objet l'utilisation d'un polynucléotide isolé, pour la détection de *Xanthomonas axonopodis* pv. *allii,* ce polynucléotide isolé, susceptible d'être obtenu à partir de *Xanthomonas axonopodis* pv. *allii,* est défini par la séquence SEQ ID NO :6 et est choisi dans le groupe constitué par :
a) le polynucléotide de séquence SEQ ID NO: 6 ;
b) un polynucléotide ayant 85 %, de préférence 90 %, et plus préférentiellement 95 % d'identité avec la séquence SEQ ID NO : 6 ;
c) tout fragment d'au moins 15 pb dudit polynucléotide ;
d) tout polynucléotide complémentaire de l'un des polynucléotides a) ou b);
e) tout polynucléotide capable de s'hybrider sélectivement, en conditions stringentes avec l'un des polynucléotides a), b), c) ou d).

Les pourcentages d'identité auxquels il est fait référence dans le cadre de l'exposé de la présente invention sont déterminés sur un alignement global des séquences à comparer, en utilisant l'algorithme de NEEDLEMAN et WUNSCH (J. Mol. Biol. 48, 443-453, 1970). Cette comparaison de séquences peut être effectuée par exemple à l'aide du logiciel d'alignement « Geneious » (Drummond AJ, Ashton B, Cheung M, Heled J, Kearse M, Moir R, Stones-Havas S, Thierer T, Wilson A ; 2009, Geneious v4.7).

Des conditions stringentes d'hybridation, pour un polynucléotide donné, peuvent être identifiées par l'homme du métier en fonction de la taille et de la composition en bases du polynucléotide concerné, ainsi que de la composition du mélange d'hybridation (notamment pH et force ionique). Généralement, des conditions stringentes, pour un polynucléotide de taille et de séquence données, sont obtenues en opérant à une température inférieure d'environ 5°C à 10°C à la température de fusion (Tm) de l'hybride formé, dans le même mélange réactionnel, par ce polynucléotide et son complémentaire.

La présente invention englobe notamment des polynucléotides utilisables comme amorces d'amplification pour l'obtention d'une séquence d'acide nucléique conforme à l'invention, ou comme sondes d'acide nucléique pour la détection de ladite séquence.

Elle englobe en particulier les couples d'amorces utilisables pour l'amplification d'un polynucléotide de séquence SEQ ID NO: 6, ou d'un fragment de celui-ci.

A titre d'exemples non-limitatifs de couples d'amorces conformes à l'invention, on citera notamment :
- le couple d'amorces constitué par les polynucléotides PXaa2U (SEQ ID NO: 7) et PXaa2L, (SEQ ID NO: 8), qui permet l'amplification d'un fragment de 995 pb de la séquence SEQ ID NO: 6 :
- le couple d'amorces constitué par les polynucléotides NXaa2U (SEQ ID NO: 9) et NXaa2L, (SEQ ID NO: 10), qui permet l'amplification d'un fragment de 401 pb de la séquence SEQ ID NO: 6.

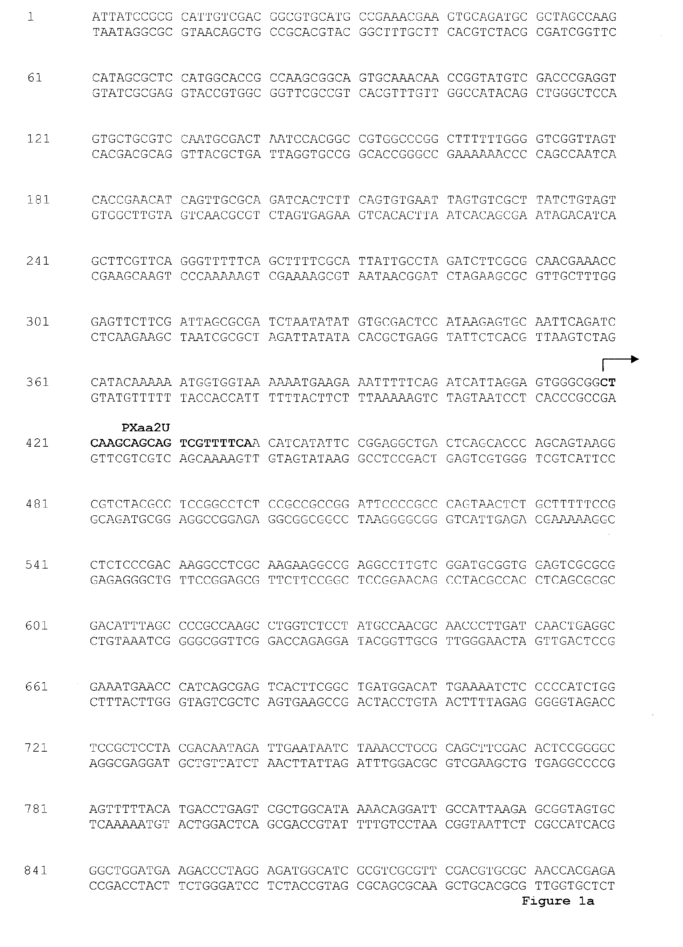

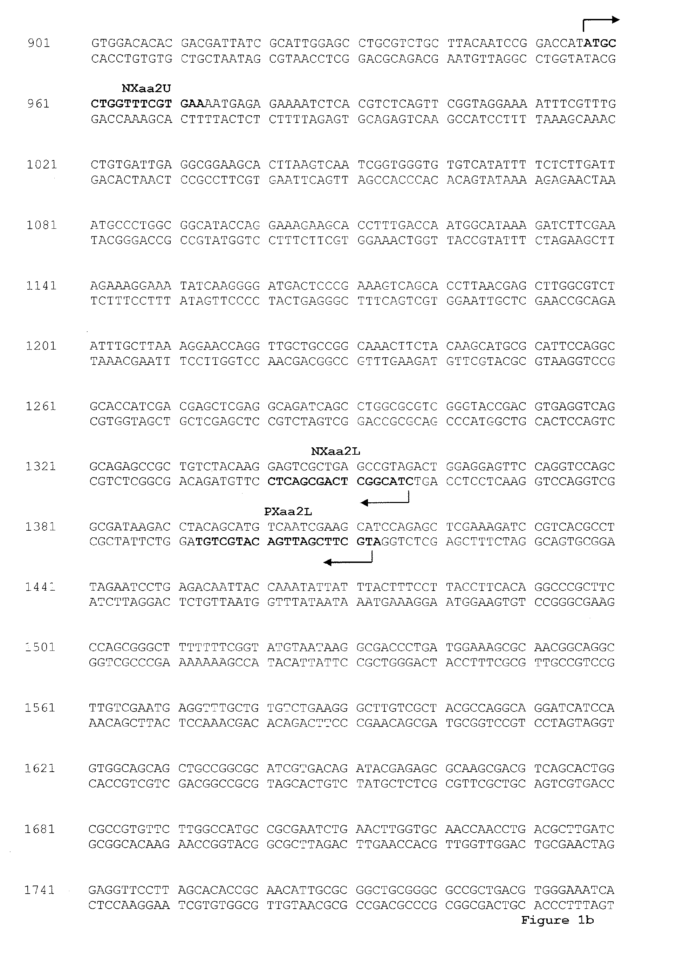

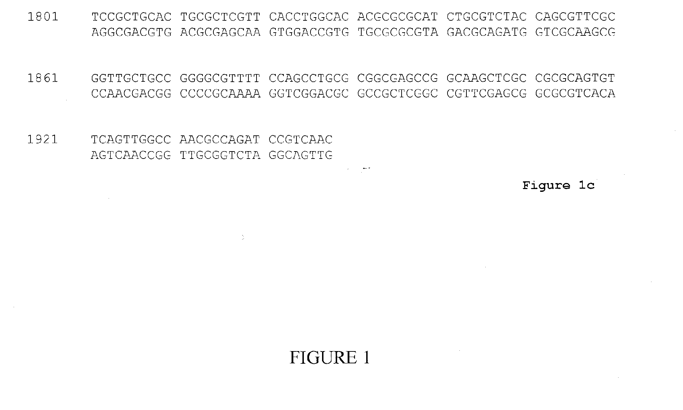

La Figure 1 représente la séquence SEQ ID NO : 6. Les fragments de séquence en gras délimitent les amorces PXaa2U, PXaa2L, NXaa2U et NXaa2L.

Un autre objet de la présente invention est l'utilisation de molécules d'acide nucléique conformes à l'invention pour le dépistage de *Xanthomonas axonopodis* pv. *allii,* et notamment les souches qui ne comprennent pas le marqueur « PIL ».

La présente invention a également pour objet un procédé de dépistage de *Xanthomonas axonopodis* pv. *allii,* caractérisé en ce qu'il comprend :
- la mise en présence d'ADN d'un échantillon biologique susceptible de contenir ladite bactérie avec un ou plusieurs polynucléotide(s) conforme(s) à l'invention, en conditions permettant une hybridation sélective entre ledit ou lesdits polynucléotide(s) et la séquence cible SEQ ID NO: 6, si celle-ci est présente dans ledit ADN ;
- la détection de ladite hybridation.

L'échantillon biologique utilisé peut être soit une culture de bactéries isolées à partir de la plante ou des graines d'alliacées sur lesquelles on souhaite opérer la détection, soit directement les graines d'alliacées potentiellement infectées, ou un échantillon de la plante potentiellement infectée. Avantageusement, la détection est réalisée sur les graines d'oignon.

La détection de l'hybridation peut s'effectuer par tous moyens connus en eux-mêmes de l'homme de l'art. Lorsque l'on utilise un polynucléotide conforme à l'invention, préalablement marqué à l'aide d'un marqueur approprié, en tant que sonde d'acide nucléique, l'hybridation de la séquence cible avec la sonde marquée est détectée directement.

Lorsque l'on utilise deux polynucléotides, constituant un couple d'amorces conformes à l'invention, l'hybridation des amorces avec la séquence cible est mise en évidence par une réaction d'amplification en chaîne par polymérase (amplification PCR pour « Polymerase chain reaction »), puis par détection du produit d'amplification, notamment sur gel d'agarose.

Dans ce cas, le procédé de dépistage conforme à l'invention comprend au moins les étapes suivantes :
i) la mise en présence de l'ADN d'un échantillon biologique à tester avec un couple d'amorces conformes à l'invention, en conditions permettant une hybridation sélective entre les amorces et la séquence cible SEQ ID NO: 6 ;
ii) la réalisation d'une réaction d'amplification en chaîne par polymérase dans des conditions permettant l'amplification de la séquence cible SEQ ID NO: 6 ;
iii) la détection du produit d'amplification.

De manière préférée, la détection est réalisée par PCR « nichée » (ou nested PCR). Dans ce mode de mise en oeuvre de l'invention, le procédé de dépistage conforme à l'invention comprend en outre une seconde étape d'amplification PCR (étape iv) réalisée à l'aide d'un second couple d'amorces permettant l'amplification d'un fragment interne du produit d'amplification de la première étape.

A titre d'exemple, la première étape d'amplification (étape ii) du procédé conforme à ce mode de mise en oeuvre peut être effectuée en utilisant le couple d'amorces constitué par les polynucléotides PXaa2U (SEQ ID NO: 7) et PXaa2L, (SEQ ID NO: 8), et la deuxième étape d'amplification (étape iv) en utilisant le couple d'amorces constitué par les polynucléotides NXaa2U (SEQ ID NO: 9) et NXaa2L (SEQ ID NO: 10).

De manière à pouvoir détecter toutes les souches de *Xanthomonas axonopodis* pv. *allii,* c'est-à-dire les souches qui contiennent les marqueurs « PIL » et « AVR », et celles qui ne contiennent qu'un de ces deux marqueurs, un autre mode de mise en oeuvre particulièrement avantageux de la présente méthode permet la détection de la séquence cible SEQ ID NO : 5 (marqueur « PIL ») outre celle de SEQ ID NO : 6 (marqueur « AVR »).

Dans ce mode de mise en oeuvre particulier, on utilise un ou plusieurs polynucléotide(s) conforme(s) à l'invention capables de s'hybrider sélectivement avec la séquence cible SEQ ID NO : 6, et un ou plusieurs polynucléotide(s) capable(s) de s'hybrider sélectivement, en conditions stringentes, avec la séquence cible SEQ ID NO : 5.

Dans le cas où le procédé de détection selon l'invention comprend une étape d'amplification PCR, on utilise des couples d'amorces permettant l'amplification d'un polynucléotide de séquence SEQ ID NO: 5, ou d'un fragment de celui-ci, et des amorces conformes à l'invention permettant l'amplification d'un polynucléotide de séquence SEQ ID NO: 6, ou d'un fragment de celui-ci.

Dans ce cas, à l'étape i) du procédé conforme à l'invention, l'ADN de l'échantillon est en outre mis en présence d'un ou plusieurs polynucléotide(s) capable(s) de s'hybrider sélectivement, en conditions stringentes, avec la séquence cible SEQ ID NO : 5, et les conditions de l'étape ii) permettent, outre l'amplification de la séquence cible SEQ ID NO: 6, l'amplification de la séquence cible SEQ ID NO: 5.

De préférence, les couples d'amorces utilisables pour l'amplification du polynucléotide de séquence SEQ ID NO: 5 sont :
- le couple d'amorces PXaa1U (SEQ ID NO: 1) et PXaa1L (SEQ ID NO: 2), qui permet l'amplification d'un fragment de 694 pb de la séquence SEQ ID NO: 5 ; et
- le couple d'amorces NXaa1U (SEQ ID NO: 3) et NXaa1L (SEQ ID NO: 4), qui permet l'amplification d'un fragment de 444 pb de la séquence SEQ ID NO: 5.

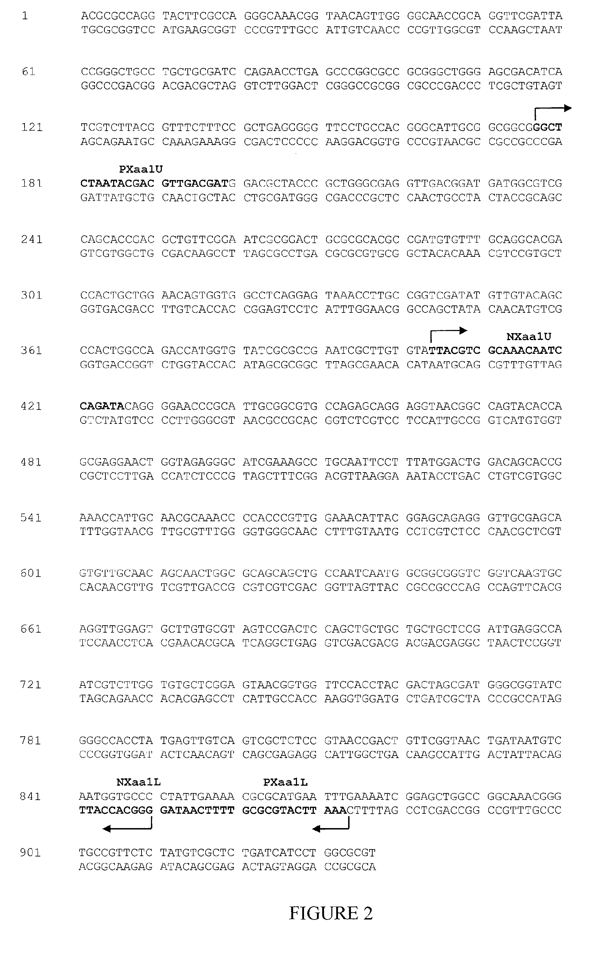

La Figure 2 représente la séquence SEQ ID NO : 5. Les fragments de séquence en gras délimitent les amorces PXaa1U, PXaa1L, NXaa1U et NXaa1L.

Lorsqu'il s'agit d'une méthode de détection selon l'invention comprenant deux étapes d'amplification (mode de mise oeuvre « PCR nichée »), on utilise lors de la première étape d'amplification (étape ii)) un couple d'amorces permettant l'amplification de tout ou partie du polynucléotide de séquence SEQ ID NO: 5, outre le couple d'amorces selon l'invention permettant l'amplification de tout ou partie du polynucléotide de séquence SEQ ID NO: 6. Dans la seconde étape d'amplification PCR (étape iv), on utilise un couple d'amorces permettant l'amplification d'un fragment interne du produit d'amplification de la séquence SEQ ID NO : 5 obtenu à la première étape, outre les amorces selon l'invention permettant d'amplifier un fragment interne du produit d'amplification de la séquence SEQ ID NO : 6.

Dans un mode de réalisation particulièrement avantageux du procédé selon l'invention, l'étape d'amplification ii) est réalisée avec le couple d'amorces PXaa1U (SEQ ID NO: 1) et PXaa1L (SEQ ID NO: 2) et le couple d'amorces PXaa2U (SEQ ID NO: 7) et PXaa2L (SEQ ID NO: 8), l'étape d'amplification iv) est réalisée avec le couple d'amorces NXaa1U (SEQ ID NO: 3) et NXaa1L (SEQ ID NO: 4) et le couple d'amorces NXaa2U (SEQ ID NO: 9) et NXaa2L (SEQ ID NO: 10). Ce mode de réalisation a un seuil de sensibilité d'environ 1 graine contaminée sur 27 300 : il est donc particulièrement fiable pour évaluer l'état sanitaire d'un échantillon de graines, et est utilisable pour la certification de semences d'alliacées puisque le taux de contamination des semences rapporté pour les épidémies de dépérissement bactérien de l'oignon en milieu tropical est de 4,5/10000.

Les modes de mise en oeuvre du procédé selon l'invention ciblant à la fois les gènes cibles « PIL » et « AVR » ont l'avantage de pouvoir détecter l'ensemble des souches de *Xanthomonas axonopodis* pv. *allii,* comme l'ont démontré les Inventeurs sur une « collection mondiale », répertoriée dans le Tableau I (cf. ci-après Exemple 2.1), constituée par 87 souches provenant de différentes régions du monde, alors que la plupart des souches de *Xanthomonas* différentes du pathovar *Xanthomonas axonopodis* pv. *allii* ne sont pas détectées. Seules quelques souches classifiées dans les sous-groupes génétiques 9.1 et 9.2 de *Xanthomonas axonopodis,* non pathogènes pour l'oignon, sont également détectées. Cependant, ces dernières peuvent facilement être distinguées des souches de *Xanthomonas axonopodis* pv. *allii* par une analyse du profil de restriction, par exemple en utilisant l'enzyme *Nhe*I qui clive uniquement le produit d'amplification des souches de *Xanthomonas euvesicatoria* (groupe 9.2), ou en utilisant l'enzyme *Cfr*I qui clive uniquement le produit d'amplification de *Xanthomonas axonopodis* sous-groupe 9.1, notamment *Xanthomonas axonopodis* pv. *begoniae.*

La présente invention a également pour objet un kit de détection de *Xanthomonas axonopodis* pv. *allii* caractérisé en ce qu'il comprend un ou plusieurs polynucléotide(s) conforme(s) à l'invention capable(s) de s'hybrider sélectivement en conditions stringentes avec la séquence cible SEQ ID NO: 6 et un ou plusieurs polynucléotide(s) capable(s) de s'hybrider sélectivement en conditions stringentes avec la séquence cible SEQ ID NO: 5.

Dans le cas de la mise en oeuvre d'un dépistage par PCR nichée sur les marqueurs « PIL » et « AVR », le kit de détection conforme à l'invention comprend les couples d'amorces d'amplification suivants :
- le couple d'amorces constitué par les polynucléotides PXaa1U (SEQ ID NO : 1) et PXaa1L (SEQ ID NO : 2) ;
- le couple d'amorces constitué par les polynucléotides NXaa1U (SEQ ID NO : 3) et NXaa1L (SEQ ID NO : 4) ;
- le couple d'amorces constitué par les polynucléotides PXaa2U (SEQ ID NO : 7) et PXaa2L (SEQ ID NO : 8) ; et
- le couple d'amorces constitué par les polynucléotides NXaa2U (SEQ ID NO : 9) et NXaa2L (SEQ ID NO : 10).

La présente invention sera mieux comprise à l'aide du complément de description qui va suivre, qui se réfère à des exemples non-limitatifs illustrant la mise en oeuvre de la présente invention pour la détection de *Xanthomonas axonopodis* pv. *allii.*

### EXEMPLE 1 : IDENTIFICATION DES MARQUEURS « PIL » ET « AVR ».

### 1.1. Identification du marqueur « PIL »

Les souches utilisées lors de la présente étude ont été cultivées sur milieu YPGA (7 g.l⁻¹ d'extrait de levure, 7 g.l⁻¹ de peptone, 7 g.l⁻¹ de glucose, 18 g.l⁻¹ d'agar, pH 7,2) ou sur milieu de Wilbrink modifié (10 g.l⁻¹ saccharose, 5 g.l⁻¹ bacto-peptone, 5 g.l⁻¹ d'extrait de levure, 0,5 g.l⁻¹ de K₂HPO₄, 0,25 g.l⁻¹ de MgSO₄/7H₂O, 0,05 g.l⁻¹ de Na₂SO₃, 15 g.l⁻¹ d'agar, pH 6,9-7 ; Rott et al., Agron. Trop. ; 43 : 244-251, 1988) pour les souches présentant une croissance faible sur le milieu YPGA.

L'amplification aléatoire d'ADN polymorphe, ou RAPD, a ensuite été réalisée. Pour cela, l'ADN total de vingt-deux souches de *Xanthomonas axonopodis* pv. *allii* provenant de diverses zones géographiques (cf. plus bas, Tableau I, les souches marquées par « ^{b} ») et de six autres *Xanthomonas* (cf. plus bas, Tableau II, les souches marquées par « ^{d} ») a été extrait à partir de 2 ml de culture bactérienne en utilisant le kit « DNeasy blood and tissue » (Qiagen, Courtaboeuf, France) selon les instructions du fournisseur.

Une amplification PCR a été effectuée sur les extraits d'ADN issus de chaque souche. Le milieu réactionnel de 25µl contient 25 ng d'ADN génomique bactérien, 3mM de MgCl₂, 0,4 µM d'amorces, 2,5 unité d'ADN polymérase Taq (Invitrogen, Merelbeke, Belgique), 100µM de chacun des dNTP (Roche Diagnotics, Meylan, France) en milieu tris-HCl 20mM et 50mM de tampon KCl (pH 8,4). Une centaine d'amorces courtes de 10 nucléotides de séquence définie arbitrairement provenant des kits 80, 70 et 60% GC (Genome Express, Meylan, France) a été testée.

L'amplification a été conduite dans les conditions suivantes :
- dénaturation initiale à 94°C pendant 7 min,
- 40 cycles d'amplification comprenant chacun 3 phases : 94°C pendant 1 min (dénaturation), 35°C pendant 1 min (hybridation des amorces sur l'ADN) et 72°C pendant 2 min (polymérisation),
- extension à 72°C pendant 5 min.

Les produits d'amplification ont été soumis à électrophorèse en gel d'agarose NuSieve 2% et révélés par fluorescence après coloration au bromure d'éthidium.

Il s'est avéré qu'aucun des fragments n'était présent dans l'ensemble des souches *Xanthomonas axonopodis* pv. *allii* testées et absent des autres pathovars. Parmi les fragments amplifiés retrouvés dans la plupart des souches de *Xanthomonas axonopodis* pv. *allii* et absents des autres souches, le fragment amplifié par l'amorce nommée 80-21 (SEQ ID NO : 11 ; 5'ACGCGCCAGG 3'), d'environ 940 pb, est présent dans 70% des souches de *Xanthomonas axonopodis* pv. *allii* testées.

Ce fragment d'intérêt a été excisé du gel d'agarose pour les souches *Xanthomonas axonopodis* pv. *allii* CFBP6364, CFBP6366, CFBP6369 et CFBP6379, extrait en utilisant le kit d'extraction « QIAquick gel Extraction » (Qiagen, Courtaboeuf, France), puis cloné par ligation dans le vecteur pGEM-T Easy selon les instructions du fournisseur (Promega, Madison, USA). La séquence a ensuite été déterminée par séquençage en utilisant les amorces T7 et SP6 qui s'hybrident aux régions bordant l'insert cloné dans le vecteur pGEM-T Easy. Ce fragment est identique à 99% entre ces quatre souches, et une recherche dans la base de données GenBank révèle qu'il partage 97% d'identité avec deux portions contiguës (respectivement de 828 et 102 pb) appartenant aux gènes PILW et PILX de *Xanthomonas euvesicatoria*, gènes qui codent pour des protéines d'assemblage du pilus.

Il est à noter qu'aucun des autres fragments identifiés par RAPD n'était présent chez les souches dont le génome n'était pas amplifié par l'amorce 80-21.

### 1.2. Identification du marqueur « AVR »

Les souches *Xanthornaonas axonopodis* pv. *allii* CFBP6369, CFBP6380, CFBP6384, JX36-1, CFBP6386 et CFBP6382, représentatives des souches non amplifiées par l'amorce 80-21 ont été analysées par la technique AFLP. Une souche de *Xanthomonas citri pv. citri* (IAPAR 306) et de *Xanthomonas campestris pv. campestris* (CFBP 5251) ont été utilisées comme contrôle.

L'analyse AFLP a été réalisée comme décrit par Ah-You et al. (International Journal of Systematic and Evolutionary Microbiology, 59 : 306-318, 2009).

L'ADN bactérien, extrait comme indiqué précédemment, a été digéré conjointement par *Sac*I et *Msp*I, puis la ligation avec les adapateurs *Sac*I et *Msp*I a été réalisée durant 3 heures à 37°C en ajoutant 2,5 µl de produit de digestion à 22,5 µl d'un mélange de ligation contenant 2 µM de l'adaptateur *Msp*I, 0,2 µM de l'adaptateur *Sac*I (Applied Biosystems, Courtaboeuf, France) et 2 unités d'ADN ligase T4 (New England Biolabs Ozyme) dans du tampon de ligation 1X. Une pré-amplification PCR est réalisée dans 15 µl de mélange réactionnel constitué de 5 µl du mélange de ligation dilué au dixième, 2,5 mM de MgCl₂, 0,23 µM de chacune des amorces PSAC (SEQ ID NO : 12) et PMSP (SEQ ID NO : 13), 0,45 µM de chaque dNTP, 0,5 unité d'ADN polymérase Taq (Goldstar Red, Eurogentec, Seraing, Belgique) dans un tampon 1X Goldstar. L'amplification a eu lieu dans les conditions suivantes :
- extension initiale à 72°C pendant 2 min,
- dénaturation initiale à 92°C pendant 2 min,
- 25 cycles d'amplification comprenant chacun 3 phases : 94°C pendant 30 sec (dénaturation), 56°C pendant 30 sec (hybridation des amorces sur l'ADN) et 72°C pendant 2 min (polymérisation),
- extension à 72°C pendant 10 min.

Les produits d'amplification sont ensuite dilués 10 fois dans de l'eau HPLC avant l'amplification sélective. L'amplification sélective est réalisée à l'aide des paires d'amorces non marquées *Msp*I (SEQ ID NO : 14) + A, T, G ou C et *Sac*I + C (SEQ ID NO : 15), ou *Msp*I (SEQ ID NO : 14) + A, T, G ou C et *Sac*I + CT (SEQ ID NO : 16). Les conditions d'amplification sont les mêmes que celles décrites pour la pré-amplification, à l'exception de la concentration des amorces *Sac*I + C et *Sac*I + CT qui est de 0,12 µM.

Les produits d'amplification ont ensuite été analysés sur gel d'acrylamide 5% (Risterucci et al., Theor. Appl. Genet.; 101 : 948-955, 2000) puis l'ADN visualisé par coloration à l'argent (Qu et al., Electrophoresis ; 26 : 99-101, 2005). Du fait de la complexité des profils AFLP obtenus en utilisant deux bases sélectives dans l'étape d'amplification, seuls les profils obtenus avec trois bases sélectives ont été exploités pour récupérer les fragments d'intérêt.

Deux fragments présents dans la majorité des souches de *Xanthomonas axonopodis* pv. *allii,* dont les souches qui ne sont pas amplifiées par l'amorce 80-21, ont été visualisés.

Afin d'identifier ces fragments, l'ADN a été extrait du gel d'acrylamide dans 50 µl de tampon. 5 µl ont ensuite été soumis à une amplification sélective, comme décrit par Ah-You et al. (International Journal of Systematic and Evolutionary Microbiology, 59 : 306-318, 2009), en utilisant les paires d'amorces *Msp*I (SEQ ID NO : 14) + A ou T et *Sac*I + CT (SEQ ID NO : 16). Les conditions d'amplification sont les mêmes que celles décrites pour la pré-amplification, à l'exception de la concentration de l'amorce *Sac*I + CT qui est de 0,12 µM.

Les fragments amplifiés ont alors été introduits par ligation dans le vecteur pGEM-T Easy comme décrit ci-dessus, puis des bactéries E. *coli* ont été transformées par les vecteurs contenant les inserts. Malgré les conditions plus stringentes d'amplification avec trois bases sélectives, le séquençage des clones obtenus a révélé que les ADN avaient une composition hétérogène, ce qui s'explique sans doute par une co-migration de produits d'amplification de taille similaire ou une contamination lors de l'étape d'excision. Néanmoins, en utilisant l'amplification avec trois nucléotides sélectifs, un clone intéressant a été obtenu à partir des fragments AFLP provenant de la souche CFBP 6382. Le séquençage de l'insert de ce clone suivi d'une recherche dans la base de données GenBank ont révélé la présence d'une séquence de 270 pb possédant 90% d'identité avec une portion du gène d'avirulence avrRxv de *Xanthomonas euvesicatoria* (numéro d'accession GenBank L20423). A partir de cette séquence, les amorces F1154 (SEQ ID NO : 17) et R1391 (SEQ ID NO : 18) ont été générées puis une PCR a été réalisée à l'aide de ces amorces sur les souches CFBP6366, CFBP6380, CFBP6384, JX36-1, CFBP 6386, CFBP6357 et CFBP6382.

L'amplification PCR a été réalisée dans 25 µl de mélange réactionnel constitué de 20 ng d'ADN total, 3mM de MgCl₂, 0,2 µM d'amorces, 1,25 unité de polymérase Dap Goldstar (Eurogentec, Seraing, Belgique), 100µM de chacun des dNTP (Roche Diagnotics, Meylan, France). Les conditions d'amplification suivantes ont été utilisées:
- extension initiale à 72°C pendant 2 min,
- dénaturation initiale à 94°C pendant 5 min,
- 40 cycles d'amplification comprenant chacun 3 phases : 95°C pendant 1 min (dénaturation), 60°C pendant 1 min (hybridation des amorces sur l'ADN) et 72°C pendant 2 min (polymérisation),
- extension à 72°C pendant 5 min.

Un amplicon de 238 pb a été obtenu pour chacune des souches testées : suivant les souches, le fragment de 238 pb partageait 90 à 99% du gène d'avirulence avrRxv de *Xanthomonas euvesicatoria* (numéro d'accession GenBank L20423). Afin d'obtenir des fragments d'ADN plus grands afin de concevoir des amorces PCR spécifiques de *Xanthomonas axonopodis* pv. *allii,* des amplifications ont été réalisées en utilisant les paires d'amorces F202 (SEQ ID NO: 19), correspondant aux nucléotides 202-219 du gène d'avirulence *avrRxv* de *Xanthornaonas euvesicatoria* (numéro d'accession GenBank L20423) et R1391 (SEQ ID NO : 18) choisie dans l'amplicon de 238 pb, ainsi que les paires d'amorces F1154 (SEQ ID NO : 17) et R2149 correspondant aux nucléotides 2131-2149 (SEQ ID NO : 20) du gène d'avirulence *avrRxv* de *Xanthornaonas euvesicatoria* (numéro d'accession GenBank L20423). Un fragment de 1948 pb a été amplifié pour les souches CFBP6366, CFBP6386, CFBP6357 et CFBP6382, un fragment de 1950 pb pour les souches CFBP 6384 et JX36-1 et une séquence partielle de 1504 pb pour la souche CFBP 6380. Toutes ces séquences présentent entre 87 et 98% d'identité avec le gène *avrRxv* de *Xanthomonas euvesicatoria*.

### EXEMPLE 2 : DÉTECTION SPÉCIFIQUE DE XANTHOMONAS AXONOPODIS PV. ALLII.

### 2.1. Spécificité de la détection par PCR nichée multiplex

A partir des marqueurs « PIL » (SEQ ID NO : 5) et « AVR » (SEQ ID NO: 6), des paires d'amorces ont été conçues en sélectionnant les régions les plus conservées de ces deux marqueurs parmi les souches de *Xanthorraonas axonopodis* pv. *allii.*

Pour le marqueur « PIL », les deux couples d'amorces choisis sont le couple d'amorces PXaa1U (SEQ ID NO: 1) et PXaa1L (SEQ ID NO: 2) et le couple d'amorces NXaa1U (SEQ ID NO: 3) et NXaa1L, (SEQ ID NO: 4).

Pour le marqueur « AVR », les deux couples d'amorces choisis sont le couple d'amorces PXaa2U (SEQ ID NO: 7) et PXaa2L (SEQ ID NO: 8), et le couple d'amorces NXaa2U (SEQ ID NO: 9) et NXaa2L (SEQ ID NO: 10).

Une analyse par PCR nichée multiplex a alors été réalisée à l'aide de ces quatre couples d'amorces sur 87 souches de *Xanthomonas axonopodis* pv. *allii* (cf. Tableau I ci-après), 101 souches bactériennes phytopathogènes (autres pathovars et espèces de *Xanthomonas,* autres genres bactériens), et 18 bactéries saprophytes de l'oignon (cf. Tableau II ci-après).

Différents types d'échantillons ont été testés, notamment de l'ADN génomique bactérien purifié, des suspensions bactériennes et des graines mélangées avec une souche bactérienne.

La première étape de l'analyse par PCR nichée multiplex a été effectuée dans un mélange réactionnel de 25 µl constitué de 0,5 à 5 µl d'échantillon à tester (selon le type d'échantillon), 3 mM de MgCl₂, 0,2 µM de chacune des amorces constituant les couples d'amorces PXaa2U (SEQ ID NO: 7) / PXaa2L (SEQ ID NO: 8) et PXaa1U (SEQ ID NO: 1) / PXaa1L (SEQ ID NO: 2), 100 µM de chaque dNTP, 1,25 unité d'ADN polymérase Taq (Goldstar Red, Eurogentec, Seraing, Belgique), dans un tampon 75 mM tris-HCl, 20mM (NH₄)₂SO₄ et 0,01% Tween 20 (pH 8,8).

Les conditions d'amplification utilisées étaient les suivantes :
- dénaturation initiale à 94°C pendant 5 min,
- 40 cycles d'amplification comprenant chacun 3 phases : 95°C pendant 1 min (dénaturation), 63°C pendant 1 min (hybridation des amorces sur l'ADN) et 72°C pendant 2 min (polymérisation),
- extension à 72°C pendant 5 min.

La seconde étape de la PCR nichée multiplex a été réalisée avec 1 à 5 µl des amplicons obtenus à la première étape d'amplification dans des conditions analogues à celles décrites pour la première étape d'amplification, à l'exception des couple d'amorces qui sont les suivant : NXaa2U (SEQ ID NO: 9) / NXaa2L (SEQ ID NO: 10) et NXaa1U (SEQ ID NO: 3) / NXaa1L (SEQ ID NO: 4) ont été utilisées. Le programme d'amplification était le suivant :
- dénaturation initiale à 95°C pendant 5 min,
- 30 cycles d'amplification comprenant chacun 3 phases : 94°C pendant 30 sec (dénaturation), 57°C pendant 30 sec (hybridation des amorces sur l'ADN) et 72°C pendant 40 sec (polymérisation),
- extension à 72°C pendant 5 min.

Les produits d'amplification ont ensuite été analysés sur gel d'agarose 1% (amplicon provenant de la première étape d'amplification) ou 3% (amplicon provenant de la seconde étape d'amplification).

Parmi les 87 souches de *Xanthomonas axonopodis* pv. *allii* testées, trois types de résultats sont obtenus lors de la première étape d'amplification :
i) deux amplicons présentant les tailles attendues, 694 pb (une portion du marqueur « PIL ») et 995 pb (une portion du marqueur « AVR ») ont amplifiés à partir de toutes les souches du Brésil, de Cuba, du Japon, de l'Ile Maurice, de l'Ile de la Réunion et de Hawaii.
ii) un seul amplicon de 694 pb (une portion du marqueur « PIL ») a été amplifié à partir de toutes les souches provenant de Géorgie du Venezuela, de quatre souches du Colorado et de deux souches provenant de l'Afrique du Sud.
iii) un seul amplicon de 995 pb (une portion du marqueur « AVR ») a été amplifié à partir de toutes les souches provenant de la Barbade, du Texas, trois souches provenant de l'Afrique du Sud et une provenant du Colorado.

Lors de la seconde étape d'amplification, les fragments suivants, issus de l'amplification interne des produits d'amplification de la première étape, ont été observés : deux fragments de 444 pb (une portion interne de l'amplicon « PIL ») et 401 pb (une portion interne de l'amplicon « AVR ») ont été obtenus pour le groupe présentant le profil i), un unique amplicon de 444 et 401 pb pour les groupes ii) et iii) respectivement.

Les résultats obtenus pour les 87 souches de *Xanthomonas axonopodis* pv. *allii* testées sont résumés dans le Tableau I ci-dessous.

En outre, la pathogénicité de ces 87 souches a été confirmée par vérification des postulats de Koch en utilisant, pour chaque souche, l'espèce-hôte dont elle provient.

**TABLEAU I**

| **Souches^{a}** | **origine** | **Hôte** | **Réponse en Multiplex nested-PCR^{f}** | |
|---|---|---|---|---|
| | | | 694 bp/ 444 bp amplicons (marqueur PIL) | 995 bp/ 401 bp amplicons (marqueur AVR) |
| CFBP 6384^{bdeh}, JX36-1^{deht}, CFBP 6386^{bdeht} | South Africa | *A*.*cepa L* | - / - | + / + |
| CFBP 6385^{bg}. JX36-2ⁱ | South Africa | A.cepa L | + / + | - / - |
| CFBP 6367^{bh}, JR512ⁱ, JR513^{hi}, JR514, JR515, CFBP 6368^{hi} | Barbados | *A.cepa* L | - / - | + / + |
| CFBP 6362^{bgh}, CFBP 6363^{gh}, JV593^{gh}, CFBP 6377^{ghj} | Brazil | *A.cepa* L | + / + | + / + |
| CFBP 6378^{b} | Brazil | *A.cepa* L | - / - | + / + |
| CFBP 6364^{bcgh}, CFBP 6365^{gh} | Cuba | *A*. *sativum* L | + / + | + / + |
| CFBP 6107*^{bgh}, CFBP 6108^{bghj} | Japan | *A. fistulosum* L | + / + | + / + |
| JR649, JR650, JR651, CFBP 6374^{gh}, JR653, JR654-1, CFBP 6374, JR653, JR654-1, JR655, CFBP 6376^{bgh}, JS959, JS960, JS961, JS962 | Mauritius | *A*.*cepa* L | + / + | + / + |
| JQ740-1, LMG 16528^{bghj}, CFBP 6366^{bceghj}, CFBP 6369^{bcdghj}, CFBP 6357^{egh}, JQ759, JR520-1, JR523-1, CFBP 6371^{gh}, CFBP 6372, CFBP 6373, CFBP 6375 | Réunion Island | *A*.*cepa* L | + / + | + / + |
| CFBP 6355^{gh}, LA261-18, JX366-2 | Réunion Island | *A. sativum* L | + / + | + / + |
| JX-373-2, LB239-18 | Reunion Island | *A. porrum* L | + / + | + / + |
| JY317^{gj}, CFBP 6379^{bcg}, JY319^{g} JY320^{g} | Colorado (USA) | | + / + | - / - |
| CFBP 6380^{bdehi} | Colorado (USA) | *A. cepa* L | - / - | + / + |
| JY274, JY275, JY276^{bgj} | Georgia (USA) | | + / + | - / - |
| LMG 577, LMG 578, LMG 579^{j} CFBP 6359^{bgh}, LMG 943, LMG 9487, LMG 9488, LMG 9489, CFBP 6360^{gh}, LMG 9491, LMG 9492, CFBP 6361^{bgh}, LMG 9494 | Hawaii (USA) | *A*.*cepa* L | + / + | + / + |
| JW200, CFBP 6381^{bhi}, JW202, CFBP 6382^{bde}, JW204, JW205, CFBP 6383^{hi} | Texas (USA) | *A.cepa* L | - / - | + / + |
| CFBP 6387^{bg}, JX722^{j}, JX724, JX725, JX726, CFBP 6338^{bg}, JX728 | Venezuela | *A.cepa* L | + / + | - / - |
| ^{a}CFBP. Collection Française de Bactéries Phytopathogènes, Station de Pathologie Végétale, Angers, France. BCCMTM/LMG, Bacteria collection, Laboratorium voor Microbiologie, Universiteit Gent, Belgium: Les autres souches appartiennent à notre collection de laboratoire (3P, Réunion. France). | | | | |
| Toutes les souches listées dans ce tableau sont pathogènes de l'oignon. | | | | |
| ^{b} Souches utilisées pour évaluer les marqueurs RAPD (22 souches). | | | | |
| ^{c} Souches pour lesquelles le marqueur RAD 80-21 a été cloné et séquencé (4 souches). | | | | |
| ^{d} Souches utilisées pour évaluer les marqueurs AFLP (6 souches). | | | | |
| ^{e} Souches pour lesquelles le fragment AFLP de 238 pb a été cloné et séquencé (7 souches) | | | | |
| ^{f}+. fragment de taille attendue amplifié: -, pas d'amplicon détectable. | | | | |
| ^{g} Souches pour lesquelles l'ADN a été amplifiés par les amorces PXaa1U et PXaa1L et séquencé (27 souches). | | | | |
| ^{h} Souches pour lesquelles l'ADN a été amplifié par les amorces PXaa2U and PXaa2L primers et séquencé (28 souches). | | | | |
| ⁱ Souches utilisées pour l'analyse de restriction avec CfrI (8 souches). | | | | |
| ^{j} Souches utilisées pour l'analyse de restriction avec NheI (10 souches) | | | | |
| * Souche Pathotype | | | | |

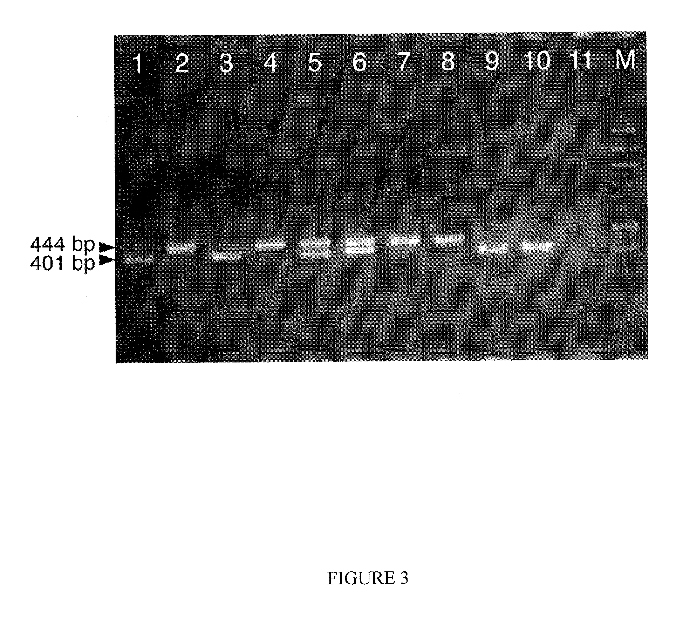

Un exemple de résultat de PCR nichée multiplex est illustré à la Figure 3. La piste 11 représente le contrôle PCR négatif, les pistes 1 à 10 représentent les résultats obtenus pour les souches CFBP 6384, CFBP 6385, CFBP 6380, CFBP 6379, CFBP 6369, CFBP 6107, JY 276, CFBP 6387, CFBP 6368 et CFBP 6381. La piste M correspond aux marqueurs de taille moléculaire.

Lors des tests de PCR nichée multiplex réalisés sur les souches saprophytes de l'oignon et sur des souches appartenant à d'autres genres bactériens, aucun produit d'amplification n'a été observé. De même, la majorité des souches de *Xanthomonas* ne sont pas amplifiées par PCR nichée multiplex. Cependant, il s'est avéré que quelques souches de *Xanthomonas axonopodis* classifiées dans le sous-groupe génétique 9.2 *sensu* Rademaker étaient également détectées. En fonction du pathovar, les amplicons du marqueur « PIL » et/ou celui du marqueur « AVR » sont mis en évidence. En outre, les amplicons du marqueur « AVR » sont également détectés pour les souches de *Xanthomonas axonopodis* pv. *begoniae* (sous-groupe génétique 9.1 *sensu* Rademaker).

Les résultats obtenus pour les souches saprophytes de l'oignon et les souches appartenant à d'autres genres bactériens testées sont résumés dans le Tableau II ci-dessous.

**TABLEAU II**

| | | **Réponse à la Multiplex nested-PCR ^{c}** | |
|---|---|---|---|
| **Souches^{a}** | **Taxon** | 694 bp/ 444 bp amplicons (marqueur PIL) | 995 bp/ 401 bp amplicons (marqueur AVR) |
| **Sous-groupe génétique 9.2** | | | |
| LMG 667^{bdefh}, LMG 668^{efh}, LMG 905^{h}. LMG 909, LMG 910^{efh}, LMG 913^{beh}, LMG 914^{h}, LMG 922^{efh}, LMG 926^{e}, LMG927^{e}, LMG 929^{eth}, LMG 930^{ef}, LMG 931^{eth}, LMG 932^{efh}, LMG 933^{efh}, CFBP 5600*^{eth} | *Xanthomonas euvesicatoria* | + / + | + / + |
| JJ238-20, JJ238-26, JJ238-27, JJ238-28 | *Xanthomonas euvesicatoria*pv. *citrumelo* | - / - | - / - |
| CFBP 2910^{bf} | *Xanthomonas axonopodis* pv. *ctriumelo* | - / - | + / + |
| LMG 497^{d*}, LMG 8019 | *Xanthomonas axonopodis* pv. *alfalfae* | - / - | - / - |
| LMG 675^{d*} | *Xanthomonas axonopodis* pv. *cassiae* | - / - | - / - |
| LMG 8048^{be} | *Xanthomonas axonopodis* pv. *cassavae* | + / + | - / - |
| LMG 861^{d*} | *Xanthomonas axonopodis* pv. *ricini* | - / - | - / - |
| LMG 686^{*} | *Xanthomonas axonopodis* pv. *coracanae* | - / - | - / - |
| LMG 691^{*} | *Xanthomonas axonopodis* pv. *cyamopsidis* | - / - | - / - |
| LMG 692^{bf*} | *Xanthomonas axonopodis* pv. *desmodii* | - / - | + / + |
| LMG 693^{be*} | *Xanthomonas axonopodis* pv. *desmodiigangetici* | + / + | - / - |
| LMG 694^{*} | *Xanthomonas axonopodis* pv. *desmodiironmdifolii* | - / - | - / - |
| LMG 698^{*} | *Xanthomonas axonopodis* pv. *erythrinae* | - / - | - / - |
| LMG 811^{*} | *Xanthomonas axonopodis* pv. *patelii* | - / - | - / - |
| LMG 844^{bef*} | *Xanthomonas axonopodis* pv. *phyllanthi* | + / + | + / + |
| LMG 819^{*} | *Xanthomonas axonopodis* pv. *poinsettiicola* | - / - | - / - |
| LMG 955^{*e} | *Xanthomonas axonopodis* pv. *tamarindi* | + / + | - / - |
| NCPPB 938^{bef} | *Xanthomonas axonopodis* pv. *lespedezae* | + / + | + / + |
| **Sous-groupe génétique 9.1** | | | |
| LMG 55l^{bfg}, LMG 7303^{bfg*}, CFBP 2524^{bfg} | *Xanthomonas axonopodis* pv. *begoniae* | + / + | + / + |
| **Sous-groupe génétique 9.3** | | | |
| LMG 982^{*}, LMG 539 | *Xanthomonas axonopodis* pv. *axonopodis* | - / - | - / - |
| LMG 901^{*}, LMG 8285 | *Xanthomonas axonopodis* pv. *vasculorum* | - / - | - / - |
| **Sous-groupe génétique 9.4** | | | |
| LMG 8014. JR518-3 | *Xanthomonas axonopodis* pv. *phaseoli* | - / - | - / - |
| CFBP 2603 | *Xanthomonas axonopodis* pv. *manihotis* | - / - | - / - |
| LMG 695^{d*} | *Xanthomonas axonopodis* pv. *dieffenbachiae* | - / - | - / - |
| **Sous-groupe génétique 9.5** | | | |
| C 39, JA159-1, CFBP 2525^{d*}, LAPAR 306 | *Xanthomonas axonopodis* pv. *citri* | - / - | - / - |
| LMG 548^{*} | *Xanthomonas axonopodis* pv. *bauhiniae* | - / - | - / - |
| LMG 558^{*} | *Xanthomonas axonopodis* pv. *cajani* | - / - | - / - |
| CFBP 1716^{*}, CFBP 2933 | *Xanthomonas axonopodis* pv. *mangiferaeindicae* | - / - | - / - |
| LMG 761^{*}, LMG 7429 | *Xanthomonas axonopodis pv. malvacearum* | - / - | - / - |
| **Sous-groupe génétique 9.6** | | | |
| LMG 7387 | *Xanthomonas axonopodis* pv. *cajani* | - / - | - / - |
| LMG 8752^{*} | *Xanthomonas axonopodis* pv. *vignicola* | | |
| **Autres espèces** | | | |
| LMG 733. CFBP 4925^{†} | *Xanthomonas hortorum* pv. hederae | - / - | - / - |
| LMG 904, LMG 915, LMG 911^{†} | *Xanthomonas vesicatoria* | - / - | - / - |
| CFBP 1156^{†} | *Xanthomonas hyacinthi* | - / - | - / - |
| CFBP 1976^{†} | *Xanthomonas bromi* | - / - | - / - |
| LMG 710, CFBP 2157^{†} | *Xanthomonas fragariae* | - / - | - / - |
| CFBP 5251^{†} | *Xanthomonas campestris pv*. *campestris* | | |
| CFBP 2528^{†} | *Xanthomonas arboricoia pv. juglandis* | - / - | - / - |
| CFBP 2532^{†} | *Xanthomonas oryzae pv*. *oryzae* | - / - | - / - |
| CFBP 2542^{†} | *Xanthomonas cucurbitae* | - / - | - / - |
| CFBP 2543^{†} | *Xanthomonas vasicola pv*. *holcicola* | - / - | - / - |
| CFBP 4188^{†} | *Xanthomonas cynarae* | - / - | - / - |
| CFBP 4641^{†} | *Xanthomonas sacchari* | - / - | - / - |
| LMG 673^{†} | *Xanthomonas cassavae* | - / - | - / - |
| CFBP 4644^{†} | *Xanthomonas melonis* | - / - | - / - |
| CFBP 4643^{†} | *Xanthomonas pisi* | - / - | - / - |
| CFBP 4690^{†} | *Xanthomonas codia* | - / - | - / - |
| CFBP4691^{†} | *Xanthomonas theicola* | - / - | - / - |
| CFBP 2523^{†} | *Xanthomonas albilineans* | - / - | - / - |
| LMG 797^{*} | *Xanthomonas oryzae pv*. *oryzicola* | - / - | - / - |
| LMG 892^{*} | *Xanthomonas translucens* pv. *undulosa* | - / - | - / -- |
| LMG 471^{†} | *Xanthomonas sacchari* | - / - | - / - |
| **Autres bactéries phytopathogènes** | | | |
| LMG 1222^{†} | *Burkholderia cepatia* | - / - | - / - |
| CFBP 2094 | *Pseudomonas savastanoi* pv. *savastanoi* | - / - | - / - |
| CFBP1670^{†} | *Pseudomonas savastanoï* | -/- | -/- |
| Run 145 | *Ralstonia solanacearum* phylotype III | -/- | -/- |
| Run 215 | *Ralstonia solanacearum* phylotype I | -/- | -/- |
| Run 17 | *Ralstonia solanacearum* phylotype II | -/- | -/- |
| Run 83 | *Ralstonia solanacearum* phylotype IV | -/- | -/- |
| LMG 1199^{†} | *Ralstonia eutropha* | -/- | -/- |
| LMG 2172^{†} | *Pseudomonas corrugata* | -/- | -/- |
| LMG 5093^{*} | *Pseudomonas syringae* pv. *tomato* | -/- | -/- |
| LMG 5942^{†} | *Ralstonia picketiti* | -/- | -/- |
| LMG 1794^{†} | *Pseudomonas fluorescens* | -/- | -/- |
| LMG 16206^{†} | *Pseudomonas putida* | -/- | -/- |
| LMG 2162^{†} | *Pseudomonas cichorii* | -/- | -/- |
| LMG 2129 | *Burkholderia andropogonis* | -/- | -/- |
| LMG 2804^{†} | *Erwinia chrysanthemi* | -/- | -/- |
| **Souches saprophytes isolées d'oignon** | | | |
| JS923, JS924 | *Erwinia sp.* | -/-- | -/- |
| JR593 | *Burkholderia sp.* | -/- | -/- |
| JR594-1 | *Stenotrophomonas sp.* | -/- | -/- |
| JR594-2 | *Pseudomonas sp.* | -/- | -/- |
| JR656-3 | *Klebsiella sp.* | -/- | -/- |
| JR656-5 | *Pantoea sp.* | -/- | -/- |
| JR656-6 | *Pantoea sp.* | -/- | -/- |
| JS741-1 | *Burkholderia sp.* | -/- | -/- |
| JS741-2, JS741-4, JS741-12 | *Flavimonas oryzihabitans* | -/- | -/- |
| JS741-5 | *Klebsiella sp.* | -/- | -/- |
| JS741-6 | *Pantoea agglomerans* | -/- | -/- |
| JS853 | *Pantoea sp.* | -/- | -/- |
| JS741-14 | *Enterobacter sp.* | -/- | -/- |
| JS741-15, JS741-16 | *Pseudomonas aeruginosa* | -/- | -/- |
| ^{a} CFBP. Collection Française de Bactéries Phytopathogènes. Station de Pathologie Végétale, Angers, France; BCCMTM/LMG, Bacteria collection, Laboratorium voor Microbiologie, Universiteit Gent, Belgium; NCPPB, the National Collection of Plant Pathogenic Bacteria (NCPPB, CSL, York, United Kingdom); Les autres souches appartiennent à notre collection de laboratoire (3P, Réunion. France), exceptée la souche LAPAR 306 qui a été fournie par l' IAPAR (Instituto Agronômico do Paraná. Londrina PR. Brazil) et pour laquelle le génôme complet est disponible (Da Silva, 2002). | | | |
| ^{b} Souches utilisées pour les tests de pathogénicité sur oignon (*Allium cepa* L., cv. Red Creole). | | | |
| ^{c} +. fragment amplifie de la taille attendue: -. pas d'amplicon détecté. | | | |
| ^{d} Souches utilisées pour évaluer les marqueurs RAPD (6 souches). | | | |
| ^{e} Souches pour lesquelles l'ADN a été amplifié par les amorces PXaa1U and PXaa1L et séquencé (18 souches). | | | |
| ^{f} Souches pour lesquelles l'ADN a été amplifié par les amorces PXaa2U and PXaa2L et séquencé (17 souches). | | | |
| ^{g} Souches utilisées pour l'analyse de restriction avec CfrI (3 souches) | | | |
| ^{h} Souches utilisées pour l'analyse de restriction avec NheI (12 souches) | | | |
| ⁱ Souche Type | | | |
| ^{j} Souche Pathotype. | | | |

Des tests de pathogénicité ont été réalisés pour toutes les souches du Tableau II, en utilisant le cultivar d'oignon Red Creole (Roumagnac et al., Eur. J. Plant Pathol., 106 : 867-877, 2000). Ces tests ont montré que toutes les souches qui n'appartiennent pas au pathovar *allii* bien que détectées par PCR nichée multiplex ne sont pas pathogènes pour l'oignon. En outre, ces dernières peuvent facilement être distinguées de la souche *Xanthomonas axonopodis* pv. *allii* par une analyse du profil de restriction. En effet, l'analyse des amplicons obtenus lors de la seconde étape d'amplification indique que l'enzyme de restriction *Nhe*I ne clive pas le fragment de 444 pb (amplicon du marqueur « PIL ») provenant de la souche *Xanthomonas axonopodis* pv. *allii*, alors qu'elle clive le produit d'amplification de la souche *Xanthomonas euvesicatoria,* générant deux fragments de 355 et 89 pb respectivement. De la même façon, l'enzyme de restriction *Cfr*I génère deux fragments de 343 et 58 pb à partir du produit d'amplification de 401 pb (amplicon du marqueur « AVR ») de *Xanthomonas axonopodis* pv. *begoniae,* alors qu'elle ne clive pas l'amplicon provenant de *Xanthomonas axonopodis* pv. *allii.*

### 2.2. Sensibilité de la détection par PCR nichée multiplex

La sensibilité du procédé de détection par PCR nichée multiplex a été déterminée en utilisant des suspensions bactériennes mélangées ou non avec des échantillons de graines d'oignon.

Les suspensions des souches CFBP 6366, CFBP 6385, CFBP 6367 de *Xanthomonas axonopodis* pv. *allii* ont été testées. Pour cela les suspensions contenant 1.10⁸ cfu.ml⁻¹ ont été diluées en série de 10 en 10. Des échantillons contenant 10 g de graines d'oignons saines ont été placés dans 50 ml de tampon stérile 0,01 M Sigma 7-9 (pH 7,2) (Sigma, Saint-Quentin Fallavier, France) et ont été inoculés avec des suspensions bactériennes à une concentration finale allant de 1.10¹ cfu.ml⁻¹ à 1.10⁷ cfu.ml⁻¹. Le contrôle négatif a été inoculé avec du tampon. En parallèle, les mêmes séries de dilution non mélangées avec les graines ont été analysées. Après 48 heures de macération à 4°C, les échantillons ont été d'une part ensemencés sur milieu semi-sélectif NCTM1 (Roumagnac et al., Eur. J. Plant. Pathol. ; 106 : 867-877, 2000), et d'autre part le génome bactérien a été extrait selon la méthode d'extraction alcaline rapide de Audy et al. (Phytopathology ; 86 : 361-366, 1993). 4 ml de chaque échantillon ont été centrifugés à 10 000 g durant 30 min à 4°C puis le culot a été remis en suspension dans 100 µl de NaOH 0,5 N contenant 0,5% de polyvinylpyrrolidone. 5 µl de lysat a été mélangé avec 495 µl d'une solution 20 mM Tris-HCl, pH 8,0. Pour chaque expérience, deux échantillons de 5 µl (pour les suspensions contenant au moins 1.10⁴ cfu.ml⁻¹) ou trois échantillons de 5 µl (pour les suspensions contenant moins de 1.10⁴ cfu.ml⁻¹) ont été testés en duplicate.

Lorsque les suspensions sont mélangées avec des graines d'oignons, la première étape de l'amplification permet de détecter 1.10⁶ cfu.ml⁻¹ (titre déterminé avec les suspensions correspondantes ensemencées sur milieu NCTM1). Lorsque la PCR nichée multiplex est utilisée, la limite de détection passe à environ 1.10³ cfu.ml⁻¹, bien qu'un signal est fréquemment obtenu (une ou deux fois sur trois essais) avec des suspensions contenant environs 1.10² cfu.ml⁻¹. Il est à noter que les tests équivalents réalisés sur les dilutions en séries qui n'ont pas été mélangées aux graines d'oignon ont donnés des résultats similaires.

Ces résultats montrent que la détection de *Xanthomonas axonopodis* pv. *allii* est possible dans un échantillon de graines à partir d'une concentration de 10⁶ bactéries/ml après la première étape d'amplification, et à partir d'une concentration de 10³ bactéries/ml voire 10² bactéries/ml après la seconde étape d'amplification.

### EXEMPLE 3 : DÉTECTION DE XANTHOMONAS AXONOPODIS PV. ALLII SUR DES ÉCHANTILLONS DE GRAINES INFECTÉS

La présence de *Xanthomonas axonopodis* pv. *allii* a été recherchée dans un lot de graines d'oignons récoltées sur un champ expérimental contaminé (Humeau et al., Phytopathology, 96 : 1345-1354, 2006). Le nombre d'échantillons pour chaque expérience (35 par expériences), consistant chacun en 10 g de graines, a été déterminé selon une distribution hypergéométrique afin de détecter au moins une graine contaminée sur 30 000 graines (*r* = 0,05). Chaque expérience a été réalisée en double.

Les échantillons de graines ont macéré dans 50 ml de tampon stérile 0,01 M Sigma 7-9 (pH 7,2) durant 48 heures. Le taux de contamination du lot a été déterminé d'une part par culture sur milieu NCTM1 des macéras purs ou dilués au dixième, et d'autre part par au moins deux analyses des macéras par PCR nichées multiplex après extraction alcaline réalisée comme décrit ci-dessus. Des contrôles négatifs dans lesquels l'échantillon a été remplacé par de l'eau ont été inclus dans les expériences.

Dans une autre série d'expériences, le même lot de graines a été mélangé avec des graines d'oignons saines dans une proportion 1:1,1, 1:2 et 1:12,8, puis le taux de contamination de deux, deux et trois échantillons indépendants des ces mélanges ont été analysés respectivement par culture sur milieu NCTM1 et PCR nichée multiplex dans les conditions indiquées précédemment.

La méthode d'amplification par PCR nichée étant connue pour produire des faux positifs, seuls les échantillons à partir desquels les fragments d'ADN attendus ont été détectés au moins deux fois sont considérés comme positifs. En outre, le taux de contamination déterminé à partir du comptage des boîtes ensemencées et des analyses par PCR nichée multiplex a été calculé selon la méthode décrite par Masmoudi et al. (Seed Sci. Technol. ; 22 : 407-414, 1994). La corrélation entre les taux de contamination déterminés par culture ou par PCR nichée multiplex et le ratio des dilutions graines infectées / graines saines a été calculée en appliquant le coefficient de corrélation de Pearson *r* (Pearson, K., Biometrika ; 18 : 105-117, 1926).

Les résultats de ces études sont reportés dans le Tableau III ci-dessous.

**TABLEAU III**

| | | | **Détection par Multiplex Nested-PCR** | | **Isolement sur milieu semi-sélectif** | | **Nombre de lots détectés par les 2 méthodes** |
|---|---|---|---|---|---|---|---|
| | | Nbre d'éch. de graines | TC | Nbre de lots positifs | TC | Nbre de lots positifs | |
| Lot de graines P2 1/10,000^{a} | Essai 1 | 35 | 0.00007675 | 6 | 0.00004953 | 4 | 3 |
| | Essai 2 | 35 | 0.0001213 | 9 | 0.00001183 | 1 | 1 |
| | **moyenne** | | **0.00010** | | **0.00003068** | | |
| | | | | | | | |
| Lot de graines P2 mélange avec des graines saines (1:1.1) 1/21.000^{b} | Essai 1 | 35 | 0.00003658 | 3 | 0 | 0 | 0 |
| | Essai 2 | 35 | 0.00004953 | 4 | 0.00002402 | 2 | 0 |
| | **moyenne** | | **0.000043055** | | **0.00001201** | | |
| | | | | | | | |
| Lot de graines P2 mélangé avec des graines saines (1:2) 1/30000^{b} | Essai 1 | 35 | 0.00003658 | 3 | 0 | 0 | 0 |
| | Essai 2 | 35 | 0.00003658 | 3 | 0.00001183 | 1 | 0 |
| | **moyenne** | | **0.00003658** | | **0.000005915** | | |
| | | | | | | | |
| Lot de graines P2 mélangé avec des graines saines (1:12.8) 1/138.000^{b} | Essai 1 | 35 | 0 | 0 | 0.00001183 | 1 | 0 |
| | Essai 2 | 35 | 0 | 0 | 0 | 0 | 0 |
| | Essai 3 | 35 | 0.00003658 | 3 | 0.00001183 | 1 | 0 |
| | **moyenne** | | **1.21933E-05** | | **0.0000079** | | |
| | | | | | | | |
| Graines saines | Essai 1 | 35 | 0 | 0 | 0 | 0 | |
| Essai 2 | Essai 2 | 35 | 0 | 0 | 0 | 0 | |
| ^{a} Taux de contamination des graines dérivés des analyses par multiplex Nested-PCR | | | | | | | |
| ^{b} Taux de contamination des graines théoriques obtenus en diluant le lot de graine P2 avec des graines saines | | | | | | | |

Les deux méthodes d'analyses ont permis la détection de *Xanthomonas axonopodis* pv. *allii*: six et neuf échantillons positifs ont respectivement été mis en évidence par PCR nichée multiplex, ce qui correspond à un taux de contamination moyen de 0,01%, alors que la culture sur milieu NCTM1 n'a permis de détecter que trois et quatre échantillons positifs, ce qui correspond à un taux de contamination de 0,0031%.

La série d'expériences réalisées avec le lot de graines mélangé avec des graines d'oignons saines dans une proportion 1:1,1, 1:2 et 1:12,8, correspondent respectivement à des taux de contamination théoriques de 1/21 000, 1/30 000 et 1/138 000, montre que la méthode par PCR nichée multiplex estime de façon précise les taux de contamination : en effet, la corrélation positive r entre le taux de contamination théorique et le taux de contamination déterminé expérimentalement par PCR nichée multiplex est de 0,90. A l'inverse, une telle corrélation n'a pas été observée lorsque l'analyse a été réalisée par culture sur milieu NCTM1.

A un taux de contamination déterminé expérimentalement de 1/27 300, la PCR nichée multiplex est positive à chaque essai. Pour les dilutions supérieures, notamment 1/82 000, uniquement un essai sur trois est positif.

Le niveau de contamination rapporté pour les épidémies de dépérissement bactérien de l'oignon en milieu tropical étant de 4,5/10 000, la détection par PCR nichée multiplex, avec un seuil de sensibilité de la méthode étant d'environ 1/27 300, est fiable et particulièrement bien adaptée pour certifier les lots de graines d'alliacées.

## Revendications

1. Utilisation d'un polynucléotide isolé, susceptible d'être obtenu à partir de *Xanthomonas axonopodis* pv. *allii*, défini par la séquence SEQ ID NO: 6 pour la détection de *Xanthomonas axonopodis* pv. *allii.*

2. Procédé de dépistage de *Xanthomonas axonopodis* pv. *allii*, **caractérisé en ce qu'**il comprend :
- la mise en présence d'ADN d'un échantillon biologique susceptible de contenir ladite bactérie avec un ou plusieurs polynucléotide(s) conforme(s) à l'invention, en conditions permettant une hybridation sélective entre ledit ou lesdits polynucléotide(s) et la séquence cible SEQ ID NO: 6, si celle-ci est présente dans ledit ADN ;
- la détection de ladite hybridation.

3. Procédé selon la revendication 2, **caractérisé en ce qu'**il comprend les étapes suivantes :
i) la mise en présence de l'ADN d'un échantillon biologique à tester avec un couple d'amorces conformes à l'invention, en conditions permettant une hybridation sélective entre les amorces et la séquence cible SEQ ID NO: 6 ;
ii) la réalisation d'une réaction d'amplification en chaîne par polymérase dans des conditions permettant l'amplification de la séquence cible SEQ ID NO: 6 ;
iii) la détection du produit d'amplification.

4. Procédé selon la revendication 3, **caractérisé en ce qu'**il comprend en outre une étape iv) qui est une amplification en chaîne par polymérase à l'aide d'un second couple d'amorces permettant l'amplification d'un fragment interne du produit d'amplification de l'étape ii).

5. Procédé selon une quelconque des revendications 2 à 4, **caractérisé en ce que** l'ADN de l'échantillon est en outre mis en présence d'au moins un polynucléotide capable de s'hybrider sélectivement, en conditions stringentes, avec la séquence cible SEQ ID NO : 5.

6. Procédé selon une quelconque des revendications 3 ou 4, **caractérisé en ce que** à l'étape i) l'ADN de l'échantillon est en outre mis en présence d'un ou plusieurs polynucléotide(s) capable(s) de s'hybrider sélectivement, en conditions stringentes, avec la séquence cible SEQ ID NO : 5, et **en ce que** les conditions de l'étape ii) permettent, outre l'amplification de la séquence cible SEQ ID NO: 6, l'amplification de la séquence cible SEQ ID NO: 5.

7. Procédé selon la revendication 6, **caractérisé en ce que** l'étape d'amplification iv) est réalisée en outre en présence d'un couple d'amorces permettant l'amplification d'un fragment interne du produit d'amplification de la première étape d'amplification du polynucléotide de séquence SEQ ID NO : 5.

8. Procédé selon la revendication 7, **caractérisé en ce que** l'étape d'amplification ii) est réalisée avec le couple d'amorces PXaa1U (SEQ ID NO: 1) et PXaa1L (SEQ ID NO: 2) et le couple d'amorces PXaa2U (SEQ ID NO: 7) et PXaa2L (SEQ ID NO: 8), et **en ce que** l'étape d'amplification iv) est réalisée avec le couple d'amorces NXaa1U (SEQ ID NO: 3) et NXaa1L (SEQ ID NO: 4) et le couple d'amorces NXaa2U (SEQ ID NO: 9) et NXaa2L (SEQ ID NO: 10).

9. Procédé selon une quelconque des revendications 2 à 8, **caractérisé en ce que** l'échantillon biologique est une graine d'alliacée.

10. Kit de détection de *Xanthomonas axonopodis* pv. *allii*, **caractérisé en ce qu'**il comprend un ou plusieurs polynucléotide(s) capable(s) de s'hybrider sélectivement, en conditions stringentes, avec la séquence cible SEQ ID NO : 6 et un ou plusieurs polynucléotide(s) capable(s) de s'hybrider sélectivement, en conditions stringentes, avec la séquence cible SEQ ID NO : 5.

11. Kit de détection selon la revendication 10, **caractérisé en ce qu'**il comprend les couples d'amorces d'amplification suivants :
- le couple d'amorces constitué par les polynucléotides PXaa1U (SEQ ID NO: 1) et PXaa1L (SEQ ID NO: 2) ;
- le couple d'amorces constitué par les polynucléotides NXaa1U (SEQ ID NO: 3) et NXaa1L (SEQ ID NO: 4) ;
- le couple d'amorces constitué par les polynucléotides PXaa2U (SEQ ID NO: 7) et PXaa2L (SEQ ID NO: 8) ; et
- le couple d'amorces constitué par les polynucléotides NXaa2U (SEQ ID NO: 9) et NXaa2L (SEQ ID NO: 10).

## Patentansprüche

1. Verwendung eines isolierten Polynukleotids, welches erhältlich ist aus *Xanthomonas axonopodis pv. allii*, und definiert ist durch die Sequenz SEQ ID NO: 6 zum Nachweis von *Xanthomonas axonopodis pv. allii.*

2. Verfahren zum Nachweis von *Xanthomonas axonopodis pv. allii*, **dadurch gekennzeichnet, dass** es umfasst:
- das Inkontaktbringen der DNA in einer biologischen Probe, weiche das Bakterium enthalten könnte, mit einem oder mehreren Polynukleotid(en) gemäß der Erfindung unter Bedingungen, die eine selektive Hybridisierung zwischen dem oder den Polynukleotid(en) und der Zielsequenz SEQ ID NO: 6 erlauben, wenn diese in der DNA vorhanden ist;
- das Nachweisen der Hybridisierung.

3. Verfahren gemäß Anspruch 2, **dadurch gekennzeichnet, dass** es die folgenden Schritte umfasst:
i) das Inkontaktbringen der DNA in einer zu testenden biologischen Probe mit einem Primerpaar gemäß der Erfindung unter Bedingungen, die eine selektive Hybridisierung zwischen den Primern und der Zielsequenz SEQ ID NO: 6 erlauben;
ii) die Durchführung einer Amplifikationsreaktion mittels Polymerasekettenreaktion unter Bedingungen, die die Amplifikation der Zielsequenz SEQ ID NO: 6 erlauben;
iii) das Nachweisen des Produktes der Amplifikation.

4. Verfahren gemäß Anspruch 3, **dadurch gekennzeichnet, dass** es weiterhin einen Schritt iv) umfasst, welcher eine Amplifikationsreaktion darstellt mittels Polymerasekettenreaktion mit Hilfe eines zweiten Primerpaars, das die Amplifikation eines internen Fragmentes des Produktes der Amplifikation aus Schritt ii) ertaubt.

5. Verfahren gemäß irgendeinem der Ansprüche 2 bis 4, **dadurch gekennzeichnet, dass** die DNA der Probe weiterhin mit mindestens einem Polynukleotid in Kontakt gebracht wird, welches in der Lage ist, unter stringenten Bedingungen mit der Zielsequenz SEQ ID NO: 5 selektiv zu hybridisieren.

6. Verfahren gemäß irgendeinem der Ansprüche 3 oder 4, **dadurch gekennzeichnet, dass** in Schritt i) die DNA der Probe weiterhin mit einem oder mehreren Polynukleotid(en) in Kontakt gebracht wird, welche(s) in der Lage ist/sind, unter stringenten Bedingungen mit der Zielsequenz SEQ ID NO: 5 selektiv zu hybridisieren, und dadurch, dass die Bedingungen des Schrittes ii), neben der Amplifikation der Zielsequenz SEQ ID NO: 6, die Amplifikation der Zielsequenz SEQ ID NO: 5 erlauben.

7. Verfahren gemäß Anspruch 6, **dadurch gekennzeichnet, dass** der Schritt der Amplifikation gemäß iv) weiterhin in Anwesenheit eines Primerpaares durchgeführt wird, welches die Amplifikation eines internen Fragmentes des Amplifikationsproduktes des ersten Amplifikationsschrittes des Polynukleotides mit der Sequenz SEQ ID NO: 5 erlaubt.

8. Verfahren gemäß Anspruch 7, **dadurch gekennzeichnet, dass** der Schritt der Amplifikation ii) mit dem Primerpaar PXaa1U (SEQ ID NO: 1) und PXaa1L (SEQ ID-NO: 2) und dem Primerpaar PXaa2U (SEQ ID NO: 7) und PXaa2L (SEQ ID NO: 8) durchgeführt wird, und **dass** der Schritt der Amplifikation iv) mit dem Primerpaar NXaa1U (SEQ ID NO: 3) und NXaa1L (SEQ ID NO: 4) und dem Primerpaar NXaa2U (SEQ ID NO: 9) und NXaa2L (SEQ ID NO: 10) durchgeführt wird.

9. Verfahren gemäß irgendeinem der Ansprüche 2 bis 8, **dadurch gekennzeichnet, dass** die biologische Probe ein Samenkorn eines Knoblauchgewächses ist.

10. Kit zum Nachweis von *Xanthomonas axonopodis pv. allii.*, **dadurch gekennzeichnet, dass** es ein oder mehrere Polynukleotid(e) enthält, das/die in der Lage ist/sind, unter stringenten Bedingungen mit der Zielsequenz SEQ ID NO: 6 selektiv zu hybridisieren, und ein oder mehrere Polynukleotid(e), das/die in der Lage ist/sind, unter stringenten Bedingungen mit der Zielsequenz SEQ ID NO: 5 selektiv zu hybridisieren.

11. Kit zum Nachweis gemäß Anspruch 10, **dadurch gekennzeichnet, dass** es die folgenden Primerpaare enthält:
- das Primerpaar bestehend aus den Polynukleotiden PXaa1U (SEQ ID NO: 1) und PXaa1L (SEQ ID NO: 2);
- das Primerpaar bestehend aus den Polynukleotiden NXaa1U (SEQ ID NO: 3) und NXaa1L (SEQ ID NO: 4);
- das Primerpaar bestehend aus den Polynukleotiden PXaa2U (SEQ ID NO: 7) und PXaa2L (SEQ ID NO: 8); und
- das Primerpaar bestehend aus den Polynukleotiden NXaa2U (SEQ ID NO: 9) und NXaa2L (SEQ ID NO: 10).

## Claims

1. Use of an isolated polynucleotide obtainable from *Xanthomonas axonopodis* pv. *allii*, defined by sequence SEQ ID No: 6, for detecting *Xanthomonas axonopodis* pv. *allii*.

2. Method of screening for *Xanthomonas axonopodis* pv. *allii*, **characterised in that** it comprises:
- bringing DNA of a biological sample which may contain said bacterium into contact with one or more polynucleotide(s) according to the invention under conditions permitting selective hybridisation between said polynucleotide(s) and the target sequence SEQ ID NO: 6 if it is present in said DNA;
- detecting said hybridisation.

3. Method according to claim 2, **characterised in that** it comprises the following steps:
i) bringing the DNA of a biological sample to be tested into contact with a pair of primers according to the invention under conditions permitting selective hybridisation between the primers and the target sequence SEQ ID NO: 6;
ii) carrying out a polymerase chain amplification reaction under conditions permitting amplification of the target sequence SEQ ID NO: 6;
iii) detecting the amplification product.

4. Method according to claim 3, **characterised in that** it further comprises a step iv) which is a polymerase chain amplification by means of a second pair of primers permitting amplification of an internal fragment of the amplification product from step ii).

5. Method according to any one of claims 2 to 4, **characterised in that** the DNA of the sample is further brought into contact with at least one polynucleotide capable of selectively hybridising, under stringent conditions, with the target sequence SEQ ID NO: 5.

6. Method according to either claim 3 or claim 4, **characterised in that** in step i) the DNA of the sample is further brought into contact with one or more polynucleotide(s) capable of selectively hybridising, under stringent conditions, with the target sequence SEQ ID NO: 5, and **in that** the conditions of step ii) permit, in addition to amplification of the target sequence SEQ ID NO: 6, amplification of the target sequence SEQ ID NO: 5.

7. Method according to claim 6, **characterised in that** the amplification step iv) is further carried out in the presence of a pair of primers permitting the amplification of an internal fragment of the amplification product from the first step of amplification of the polynucleotide of sequence SEQ ID NO: 5.

8. Method according to claim 7, **characterised in that** the amplification step ii) is carried out with the pair of primers PXaa1U (SEQ ID NO: 1) and PXaa1L (SEQ ID NO: 2) and the pair of primers PXaa2U (SEQ ID NO: 7) and PXaa2L (SEQ ID NO: 8), and **in that** the amplification step iv) is carried out with the pair of primers NXaa1U (SEQ ID NO: 3) and NXaa1L (SEQ ID NO: 4) and the pair of primers NXaa2U (SEQ ID NO: 9) and NXaa2L (SEQ ID NO: 10).

9. Method according to any one of claims 2 to 8, **characterised in that** the biological sample is an Alliaceae seed.

10. Kit for the detection of *Xanthomonas axonopodis* pv. *allii*, **characterised in that** it comprises one or more polynucleotide(s) capable of selectively hybridising, under stringent conditions, with the target sequence SEQ ID NO: 6 and one or more polynucleotide(s) capable of selectively hybridising, under stringent conditions, with the target sequence SEQ ID NO: 5.

11. Detection kit according to claim 10, **characterised in that** it comprises the following pairs of amplification primers:
- the pair of primers composed of the polynucleotides PXaa1U (SEQ ID NO: 1) and PXaa1L (SEQ ID NO: 2);
- the pair of primers composed of the polynucleotides NXaa1U (SEQ ID NO: 3) and NXaa1L (SEQ ID NO: 4);
- the pair of primers composed of the polynucleotides PXaa2U (SEQ ID NO: 7) and PXaa2L (SEQ ID NO: 8); and
- the pair of primers composed of the polynucleotides NXaa2U (SEQ ID NO: 9) and NXaa2L (SEQ ID NO: 10).


### SEQUENCE LISTING

<110> CIRAD
   ROBENE, Isabelle
   PRUVOST, Olivier
   LEGRAND, Delphine
<120> NOUVEAUX OUTILS POUR LA DETECTION DE XANTHOMONAS AXONOPODIS PV. ALLII
<130> MJP/EM/11-F1367-18WO
<160> 20
<170> PatentIn version 3.3
<210> 1
   <211> 23
   <212> DNA
   <213> Artificial
<220>
   <223> Primer Pxaa1U
<400> 1
   ggctctaata cgacgttgac gat 23
<210> 2
   <211> 23
   <212> DNA
   <213> Artificial
<220>
   <223> Primer Pxaa1L
<400> 2
   aaattcatgc gcgttttcaa tag 23
<210> 3
   <211> 23
   <212> DNA
   <213> Artificial
<220>
   <223> Primer Nxaa1U
<400> 3
   ttacgtcgca aacaatccag ata 23
<210> 4
   <211> 23
   <212> DNA
   <213> Artificial
<220>
   <223> Primer Nxaa1L
<400> 4
   gggcaccatt gacattatca gtt 23
<210> 5
   <211> 937
   <212> DNA
   <213> Xanthomonas axonopodis
<400> 5
   acgcgccagg tacttcgcca gggcaaacgg taacagttgg ggcaaccgca ggttcgatta 60
   ccgggctgcc tgctgcgatc cagaacctga gcccggcgcc gcgggctggg agcgacatca 120
   tcgtcttacg gtttctttcc gctgaggggg ttcctgccac gggcattgcg gcggcgggct 180
   ctaatacgac gttgacgatg gacgctaccc gctgggcgag gttgacggat gatggcgtcg 240
   cagcaccgac gctgttcgga atcgcggact gcgcgcacgc cgatgtgttt gcaggcacga 300
   ccactgctgg aacagtggtg gcctcaggag taaaccttgc cggtcgatat gttgtacagc 360
   ccactggcca gaccatggtg tatcgcgccg aatcgcttgt gtattacgtc gcaaacaatc 420
   cagatacagg ggaacccgca ttgcggcgtg ccagagcagg aggtaacggc cagtacacca 480
   gcgaggaact ggtagagggc atcgaaagcc tgcaattcct ttatggactg gacagcaccg 540
   aaaccattgc aacgcaaacc ccacccgttg gaaacattac ggagcagagg gttgcgagca 600
   gtgttgcaac agcaactggc gcagcagctg ccaatcaatg gcggcgggtc ggtcaagtgc 660
   aggttggagt gcttgtgcgt agtccgactc cagctgctgc tgctgctccg attgaggcca 720
   atcgtcttgg tgtgctcgga gtaacggtgg ttccacctac gactagcgat gggcggtatc 780
   gggccaccta tgagttgtca gtcgctctcc gtaaccgact gttcggtaac tgataatgtc 840
   aatggtgccc ctattgaaaa cgcgcatgaa tttgaaaatc ggagctggcc ggcaaacggg 900
   tgccgttctc tatgtcgctc tgatcatcct ggcgcgt 937
<210> 6
   <211> 1948
   <212> DNA
   <213> Xanthomonas axonopodis
<400> 6
   attatccgcg cattgtcgac ggcgtgcatg ccgaaacgaa gtgcagatgc gctagccaag 60
   catagcgctc catggcaccg ccaagcggca gtgcaaacaa ccggtatgtc gacccgaggt 120
   gtgctgcgtc caatgcgact aatccacggc cgtggcccgg cttttttggg gtcggttagt 180
   caccgaacat cagttgcgca gatcactctt cagtgtgaat tagtgtcgct tatctgtagt 240
   gcttcgttca gggtttttca gcttttcgca ttattgccta gatcttcgcg caacgaaacc 300
   gagttcttcg attagcgcga tctaatatat gtgcgactcc ataagagtgc aattcagatc 360
   catacaaaaa atggtggtaa aaaatgaaga aatttttcag atcattagga gtgggcggct 420
   caagcagcag tcgttttcaa catcatattc cggaggctga ctcagcaccc agcagtaagg 480
   cgtctacgcc tccggcctct ccgccgccgg attccccgcc cagtaactct gctttttccg 540
   ctctcccgac aaggcctcgc aagaaggccg aggccttgtc ggatgcggtg gagtcgcgcg 600
   gacatttagc cccgccaagc ctggtctcct atgccaacgc aacccttgat caactgaggc     660
   gaaatgaacc catcagcgag tcacttcggc tgatggacat tgaaaatctc ccccatctgg     720
   tccgctccta cgacaataga ttgaataatc taaacctgcg cagcttcgac actccggggc     780
   agtttttaca tgacctgagt cgctggcata aaacaggatt gccattaaga gcggtagtgc     840
   ggctggatga agaccctagg agatggcatc gcgtcgcgtt cgacgtgcgc aaccacgaga     900
   gtggacacac gacgattatc gcattggagc ctgcgtctgc ttacaatccg gaccatatgc     960
   ctggtttcgt gaaaatgaga gaaaatctca cgtctcagtt cggtaggaaa atttcgtttg    1020
   ctgtgattga ggcggaagca cttaagtcaa tcggtgggtg tgtcatattt tctcttgatt    1080
   atgccctggc ggcataccag gaaagaagca cctttgacca atggcataaa gatcttcgaa    1140
   agaaaggaaa tatcaagggg atgactcccg aaagtcagca ccttaacgag cttggcgtct    1200
   atttgcttaa aggaaccagg ttgctgccgg caaacttcta caagcatgcg cattccaggc    1260
   gcaccatcga cgagctcgag gcagatcagc ctggcgcgtc gggtaccgac gtgaggtcag    1320
   gcagagccgc tgtctacaag gagtcgctga gccgtagact ggaggagttc caggtccagc    1380
   gcgataagac ctacagcatg tcaatcgaag catccagagc tcgaaagatc cgtcacgcct    1440
   tagaatcctg agacaattac caaatattat ttactttcct taccttcaca ggcccgcttc    1500
   ccagcgggct ttttttcggt atgtaataag gcgaccctga tggaaagcgc aacggcaggc    1560
   ttgtcgaatg aggtttgctg tgtctgaagg gcttgtcgct acgccaggca ggatcatcca    1620
   gtggcagcag ctgccggcgc atcgtgacag atacgagagc gcaagcgacg tcagcactgg    1680
   cgccgtgttc ttggccatgc cgcgaatctg aacttggtgc aaccaacctg acgcttgatc    1740
   gaggttcctt agcacaccgc aacattgcgc ggctgcgggc gccgctgacg tgggaaatca    1800
   tccgctgcac tgcgctcgtt cacctggcac acgcgcgcat ctgcgtctac cagcgttcgc    1860
   ggttgctgcc ggggcgtttt ccagcctgcg cggcgagccg gcaagctcgc cgcgcagtgt    1920
   tcagttggcc aacgccagat ccgtcaac                                       1948
<210> 7
   <211> 21
   <212> DNA
   <213> Artificial
<220>
   <223> Primer Pxaa2U
<400> 7
   ctcaagcagc agtcgttttc a 21
<210> 8
   <211> 21
   <212> DNA
   <213> Artificial
<220>
   <223> Primer Pxaa2L
<400> 8
   atgcttcgat tgacatgctg t 21
<210> 9
   <211> 17
   <212> DNA
   <213> Artificial
<220>
   <223> Primer Nxaa2U
<400> 9
   atgcctggtt tcgtgaa 17
<210> 10
   <211> 17
   <212> DNA
   <213> Artificial
<220>
   <223> Primer Nxaa2L
<400> 10
   ctacggctca gcgactc 17
<210> 11
   <211> 10
   <212> DNA
   <213> Artificial
<220>
   <223> Primer 80-21
<400> 11
   acgcgccagg 10
<210> 12
   <211> 18
   <212> DNA
   <213> Artificial
<220>
   <223> Primer PSAC
<400> 12
   tagactgcgt acaagctc 18
<210> 13
   <211> 16
   <212> DNA
   <213> Artificial
<220>
   <223> Primer PMSP
<400> 13
   gatgagtcct gagcgg 16
<210> 14
   <211> 16
   <212> DNA
   <213> Artificial
<220>
   <223> Primer MSPI
<400> 14
   gatgagtcct gagcgg 16
<210> 15
   <211> 19
   <212> DNA
   <213> Artificial
<220>
   <223> Primer SacI+ C
<400> 15
   tagactgcgt acaagctcc 19
<210> 16
   <211> 20
   <212> DNA
   <213> Artificial
<220>
   <223> Primer SacI+CT
<400> 16
   tagactgcgt acaagctcct 20
<210> 17
   <211> 19
   <212> DNA
   <213> Artificial
<220>
   <223> Primer F1154
<400> 17
   gaccatatgc ctggtttcg 19
<210> 18
   <211> 20
   <212> DNA
   <213> Artificial
<220>
   <223> Primer R1391
<400> 18
   ccttaaggtg ctgactttcg 20
<210> 19
   <211> 18
   <212> DNA
   <213> Artificial
<220>
   <223> Primer F202
<400> 19
   attatccgcg cattgtcg 18
<210> 20
   <211> 19
   <212> DNA
   <213> Artificial
<220>
   <223> Primer R2149
<400> 20
   gttgacggat ctggcgttg 19